(19) Europäisches Patentamt
European Patent Office
Office européen des brevets

(11) Veröffentlichungsnummer : **0 283 490 B1**

(12) # EUROPÄISCHE PATENTSCHRIFT

(45) Veröffentlichungstag der Patentschrift : **08.01.92 Patentblatt 92/02**

(21) Anmeldenummer : **87906081.2**

(22) Anmeldetag : **12.09.87**

(86) Internationale Anmeldenummer : **PCT/EP87/00517**

(87) Internationale Veröffentlichungsnummer : **WO 88/02364 07.04.88 Gazette 88/08**

(51) Int. Cl.[5] : **C07C 255/50,** C07D 213/30, C07D 239/26, C09K 19/08, C09K 19/34

(54) **VERFAHREN ZUR HERSTELLUNG VON ETHANDERIVATEN.**

Die Akte enthält technische Angaben, die nach dem Eingang der Anmeldung eingereicht wurden und die nicht in dieser Patentschrift enthalten sind.

(30) Priorität : **24.09.86 DE 3632411**

(43) Veröffentlichungstag der Anmeldung : **28.09.88 Patentblatt 88/39**

(45) Bekanntmachung des Hinweises auf die Patenterteilung : **08.01.92 Patentblatt 92/02**

(84) Benannte Vertragsstaaten : **CH DE FR IT LI**

(56) Entgegenhaltungen :
**EP-A- 0 205 998**
**WO-A-85/04874**
**WO-A-87/04158**
**GB-A- 2 121 406**
**The Journal of Organic Chemistry, Vol 32, No. 2, 13 February 1967, American Chemical Society, (US), F.H. Rash et al.: "Alkylation of o-and p-tolunitriles with halides by means 1 of sodium amide in liquid ammonia", pages 372-376**
**Journal of Organometallic Echmistry, vol. 107, no. 2, 2 March 1976, Elsevier Sequoia S.A. (Laussanne, CH) E.M. Kaiser et al.: "Lithiated tolunitriles: preparation and reactions", pages 219-228**

(56) Entgegenhaltungen :
**Synthesis, International Journal of Methods in Synthetic Organic Chemistry, no. 1, January 1977, K. Takahashi et al.: "A concenient synthesis of substituted stilbenes by conensation of o- or p-tolunitrile with p-substituted benzaldehydes", pages 58-59**
**Streitwieser, Heathcock Introduction to Organic Chemistry, 3. Edition s.163-165; House Modern Synthetic Reactions, 2nd Ed., Benjamin 1972, S.547**

(73) Patentinhaber : **MERCK PATENT GESELLSCHAFT MIT BESCHRÄNKTER HAFTUNG**
**Frankfurter Strasse 250 Postfach 4119**
**W-6100 Darmstadt (DE)**

(72) Erfinder : **WÄCHTLER, Andreas**
**Goethestr. 34**
**W-6103 Griesheim (DE)**
Erfinder : **KRAUSE, Joachim**
**Samuel-Morse-Str. 14**
**W-6110 Dieburg (DE)**
Erfinder : **PAULUTH, Detlef**
**Reuterallee 44**
**W-6100 Darmstadt (DE)**

EP 0 283 490 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die Erfindung betrifft ein Verfahren zur Herstellung von Ethanderivaten der Formel II

$$R^1\text{-}(A^1\text{-}Z^1)_n\text{-}A^2\text{-}CH_2CH_2\text{-}A\text{-}Q$$

worin

| | |
|---|---|
| Q | CN oder, falls A Pyridin-2,5-diyl ist, p-$R^2$-Phenyl, |
| $R^1$ und $R^2$ | jeweils unabhängig voneinander H, eine Alkylgruppe mit 1 - 12 C-Atomen bedeutet, die geradkettig oder verzweigt sein kann, worin auch eine oder mehrere $CH_2$-Gruppen durch O-Atome und/oder -CO-Gruppen und/oder -O-CO- und/oder -CO-O- und/oder -CH=CH-Gruppen ersetzt sein können, wobei zwei Heteroatome nicht direkt miteinander verknüpft sind, |
| A | unsubstituiertes oder ein oder mehrmals durch F, Cl oder CN substituiertes 1,4-Phenylen, oder Pyridin-2,5-diyl, |
| $A^1$ | eine unsubstituierte oder durch F- und/oder Cl-Atome und/oder $CH_3$- und/oder CN-Gruppen substituierte 1,4-Cyclohexylengruppe, worin auch eine oder zwei nicht benachbarte $CH_2$-Gruppen durch O-Atome und/oder S-Atome ersetzt sein können, 1,4-Bicyclo(2,2,2)octylengruppe oder 1,4-Phenylengruppe, worin auch eine oder mehrere CH-Gruppen durch N ersetzt sein können, |
| $A^2$ | eine unsubstituierte oder durch F- und/oder Cl-Atome und/oder $CH_3$- und/oder CN-Gruppen substituierte 1,4-Cyclohexylengruppe, oder im Falle A Pyridin-2,5-diyl, $A^2$ auch eine Einfachbindung, |
| $Z^1$ | -CO-O-, -O-CO-, $-CH_2CH_2-$, $-OCH_2-$, $-CH_2O-$ oder eine Einfachbindung, |
| und | |
| n | 0, 1 oder 2 |

bedeutet, mit der Maßgabe, daß n = 0 ist, wenn $A^2$ eine Einfachbindung bedeutet, dadurch gekennzeichnet, daß man eine reaktionsfähige Methylenverbindung der Formel III

$$R^1\text{-}(A^1\text{-}Z^1)_n\text{-}A^2\text{-}CH_2\text{-}Y \qquad III$$

worin $R^1$, $A^1$, $Z^1$, n und $A^2$ die angegebene Bedeutung haben und Y ein Halogen darstellt, mit einer Verbindung der Formel I

$$CH_3\text{-}A\text{-}Q$$

worin A und Q die angegebene Bedeutung haben, in Gegenwart einer starken Base umsetzt.

Verbindungen der Formel II sind als Ausgangsmaterialien zur Synthese von Flüssigkristallen und/oder als Flüssigkristalle selbst geeignet.

Solche Ethanderivate werden bisher meist durch Reduktion einer entsprechenden Ketoverbindung oder durch Hydrierung entsprechender Vinylen-Verbindungen hergestellt. Eine endständige Cyanogruppe muß dabei meist am Schluß der Synthese durch Substitutionsreaktionen nachträglich eingeführt werden.

Da für diese Ausgangsmaterialien selbst schon aufwendige und über mehrere Stufen verlaufende Synthesewege notwendig sind, wurde dringend ein neuer Lösungsweg zur Herstellung solcher Verbindungen mit einer Ethylenbrücke benötigt.

Aufgabe der vorliegenden Erfindung war es, einen einfachen präparativen Zugang zu flüssigkristallinen Ethanderivaten zu finden.

Überraschenderweise wurde gefunden, daß die im Prinzip bekannte Alkylierung von Tolunitril, beschrieben von F.H. Rash, S. Boatman, C.R. Hauser in J. Org. Chem. 32(2), 372-5 (1967) oder E.M. Kaiser, J.D. Petty in J. Organometallic Chem., 107 (1976), 219, für die Synthese von Ethanderivaten, die als Flüssigkristallmaterialien und/oder als Zwischenstufen für die Herstellung von Flüssigkristallen geeignet sind, einen präparativ einfachen Zugang ermöglicht. Insbesondere überraschend war, daß Cyclohexylmethylderivate zur Alkylierung von Tolunitril eingesetzt werden können. Bei dieser Verbindungsklasse ist das reaktive Zentrum durch den Cyclohexanring sterisch abgeschirmt. Als Folge dieser sterischen Abschirmung tritt bei der Alkylierung von starken Basen (Lithium-, Magnesium- und Kupferorganischen Verbindungen sowie Phosphoryliden) mit Cyclohexylmethylhalogeniden überwiegend Eliminierung ein. Das ist überraschenderweise bei der Alkylierung von Tolunitril mit den entsprechenden Cyclohexylmethylhalogeniden nicht der Fall. Des weiteren ist überraschend, daß auch Verbindungen, in welchen A Pyridin- 2,5-diyl und Q p-$R^2$-Phenyl bedeuten, ebenfalls nach diesem Verfahren alkyliert werden können. Gegenstand der Erfindung ist daher ein Verfahren zur Herstellung von Ethanderivaten der Formel II, bei welchem eine reaktionsfähige Methylenverbindung der Formel III

$$R^1\text{-}(A^1\text{-}Z^1)_n\text{-}A^2\text{-}CH_2\text{-}Y \qquad III$$

worin $R^1$, $A^1$, $Z^1$, n und $A^2$ die angegebene Bedeutung haben und Y ein Halogen darstellt, mit einer Verbindung der Formel I,

$$CH_3\text{-}A\text{-}Q$$

worin A und Q die in Formel II angegebene Bedeutung haben, in Gegenwart einer starken Base umgesetzt wird.

Weiter sind für die Vertragsstaaten FR und IT Gegenstand der Erfindung die Verbindungen der Formel IV

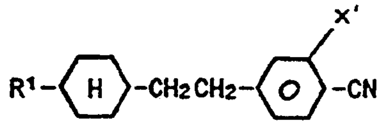

IV

worin

$R^1$ H, eine Alkylgruppe mit 1 - 12 C-Atomen bedeutet, die geradkettig oder verzweigt sein kann, worin auch eine oder mehrere $CH_2$-Gruppen durch O-Atome und/oder -CO-Gruppen und/oder -O-CO- und/oder -CO-O- und/oder -CH=CH-Gruppen ersetzt sein können, wobei zwei Heteroatome nicht direkt miteinander verknüpft sind

und

X' F, Cl oder CN bedeutet.

Das erfindungsgemäße Verfahren geht entweder von den leicht zugänglichen Tolunitrilderivaten (Formel Ia) oder von den Pyridinderivaten der Formel I d aus, die mit den reaktionsfähigen Methylenverbindungen der Formel III umgesetzt werden.

Ia

$$CH_3-A-CN$$

Id

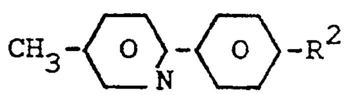

Die Umsetzung erfolgt vorzugsweise in einem inerten Lösungsmittel, das in das Reaktionsgeschehen nicht eingreift, wobei die Ausgangsverbindungen Ia bzw. I d in einem ersten Schritt in Gegenwart eines stark basischen Reagenzes, wie z. B. Lithiumdiisopropylamid, und gegebenenfalls unter Zusatz eines Reagenzes, das die Alkylierungsreaktion begünstigt wie z.B. 1,3-Dimethyltetrahydro-2-(1H)-pyrimidinon (DMPU) oder Hexamethylphosphorsäuretriamid (HMPT), in das entsprechende Carbanion überführt werden und anschließend in einem zweiten Schritt mit einer reaktionsfähigen Verbindung der Formel III alkyliert werden. Die gesamte Reaktion findet vorzugsweise bei Temperaturen von -80 bis +50 °C, insbesondere bei -70 bis 0 °C statt.

In den Formeln I, Ia und II bedeutet A beispielsweise Ringe der Formeln (1) bis (5), für Q p-$R^2$-Phenyl kommt auch der Ring (6) in Frage:

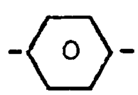

(1)

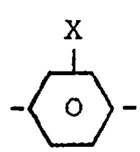

(2)

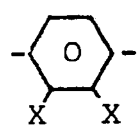

(3)

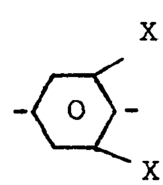

(4)

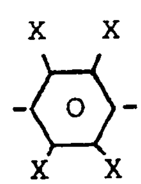

(5)

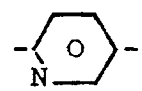

(6)

X ist darin F, Cl oder CN, insbesondere bevorzugt ist Fluor. A bedeutet vorzugsweise (1), (2), (4), (5) oder (6), insbesondere bevorzugt (1) oder (6).

Q stellt entweder eine Cyanogruppe im Falle von A Pyridin-2,5-diyl auch p-$R^2$-Phenyl dar. $R^2$ ist dann vorzugsweise eine Alkoxygruppe mit 1-10 C-Atomen, insbesondere bevorzugt Methoxy.

$R^1$ und/oder $R^2$ bedeuten bevorzugt eine Alkyl- oder Alkoxygruppe mit vorzugsweise 2-10 C-Atomen, also Ethyl, Propyl, Butyl, Pentyl, Hexyl, Heptyl, Octyl, Nonyl, Decyl, Ethoxy, Propoxy, Butoxy, Pentoxy, Hexoxy, Heptoxy, Octoxy, Nonoxy, Decoxy, ferner auch Methyl oder Methoxy. Ferner können eine oder mehrere $CH_2$-Gruppen durch -CO- und/oder -CO-O- und/oder -O-CO- und/oder -CH=CH-Gruppen ersetzt sein, wobei zwei Heteroatome nicht direkt miteinander verknüpft sind.

$R^1$ und/oder $R^2$ können auch verzweigte Reste sein wie Isopropyl, 2-Butyl, Isopentyl, 2-Hexyl, 2-Methylpentyl, 3-Methylpentyl, 2-Heptyl, 2-Octyl, Isopropoxy, Isopentoxy, 2-Butoxy oder 1-Methylpentoxy, und asymmetrische Kohlenstoffatome enthalten. In diesem Fall sind die Verbindungen der Formel II vorzugsweise optisch aktiv.

n ist 0,1 oder 2, vorzugsweise 0 oder 1.

Falls n = 0 und $A^2$ eine Einfachbindung bedeutet, dann ist $R^1$ vorzugsweise Alkyl mit 3-9, insbesondere mit 4-7 C-Atomen.

$Z^1$ hat vorzugsweise die Bedeutung einer Einfachbindung oder einer -CO-O-Gruppe, stellt aber auch $-CH_2CH_2-$, -O-CO-, $-OCH_2-$ oder $-CH_2O-$ dar.

$A^1$ bedeutet 1,4-Cyclohexylen, worin auch eine oder zwei nicht benachbarte $CH_2$-Gruppen durch O- oder S-Atome ersetzt sein können, oder eine 1,4-Phenylengruppe, worin auch eine oder mehrere CH-Gruppen durch N ersetzt sein können, stellt vorzugsweise also 1,4-Cyclohexylen, 1,3-Dioxan-2,5-diyl, 1,4-Phenylen, Pyridin-2,5-diyl oder Pyrimidin-2,5-diyl dar.

Falls $R^1$ optisch aktiv ist, oder Q p-$R^2$-Phenyl ist, kann $A^2$ auch eine Einfachbindung sein.

Y repräsentiert in Formel III ein Halogen, wie z. B. Jodide, Bromid , Chlorid .

Die Jodide sind leicht aus den entsprechenden Carbonsäuren oder deren Estern durch Reduktion z. B. mit Lithiumaluminiumhydrid und Überführung der daraus resultierenden Alkohole in die entsprechenden Mesylate und nachfolgender Finkelstein-Reaktion herstellbar.

Aus den entsprechenden Ausgangsverbindungen erhält man Ethanderivate der Formel II.

Der Einfachheit halber bedeutet im folgenden Phe eine 1,4-Phenylengruppe, Cy eine 1,4-Cyclohexylengruppe, Dio eine 1,3-Dioxan-2,5-diylgruppe, Dit eine 1,3-Dithian-2,5-diylgruppe und Pyr eine Pyrimidin-2,5-diylgruppe, wobei diese Gruppen unsubstituiert oder durch F- und/oder Cl-Atome und/oder $CH_3$-Gruppen und/oder CN-Gruppen substituiert sein können.

Verbindungen der Formel II umfassen Nitrilverbindungen der Teilformeln IIa bis IIf (mit zwei Ringen), IIg bis IIu (mit drei Ringen) und IIv bis IIaa (mit vier Ringen):

$R^1$-Cy-$CH_2CH_2$-A-CN IIa
$R^1$-Phe-$CH_2CH_2$-A-CN IIb
$R^1$-Dio-$CH_2CH_2$-A-CN IIc
$R^1$-Dit-$CH_2CH_2$-A-CN IId
$R^1$-Pyr-$CH_2CH_2$-A-CN IIe
$R^1$-Bi-$CH_2CH_2$-A-CN IIf
$R^1$-Cy-Cy-$CH_2CH_2$-A-CN IIg
$R^1$-Cy-$Z^1$-Cy-$CH_2CH_2$-A-CN IIh
$R^1$-Bi-$Z^1$-Cy-$CH_2CH_2$-A-CN IIi
$R^1$-Phe-Cy-$CH_2CH_2$-A-CN IIj
$R^1$-Phe-$Z^1$-Cy-$CH_2CH_2$-A-CN IIk
$R^1$-Cy-Phe-$CH_2CH_2$-A-CN IIl
$R^1$-Cy-$Z^1$-Phe-$CH_2CH_2$-A-CN IIm
$R^1$-Phe-Phe-$CH_2CH_2$-A-CN IIn
$R^1$-Phe-$Z^1$-Phe-$CH_2CH_2$-A-CNIIo
$R^1$-Bi-$Z^1$-Phe-$CH_2CH_2$-A-CN IIp

$R^1$-Pyr-Phe-$CH_2CH_2$-A-CN IIq
$R^1$-Dio-Cy-$CH_2CH_2$-A-CN IIr
$R^1$-Pyr-$Z^1$-Cy-$CH_2CH_2$-A-CN IIs
$R^1$-Dit-Phe-$CH_2CH_2$-A-CN IIt
$R^1$-Cy-$Z^1$-Cy-$CH_2CH_2$-A-CN IIu
$R^1$-Phe-$Z^1$-Cy-$Z^1$-Cy-$CH_2CH_2$-A-CN IIv
$R^1$-Cy-$Z^1$-Phe-$Z^1$-Phe-$CH_2CH_2$-A-CN IIw
$R^1$-Cy-$Z^1$-Phe-$Z^1$-Pyr-$CH_2CH_2$-A-CN IIx
$R^1$-Phe-$Z^1$-Dio-$Z^1$-Cy-$CH_2CH_2$-A-CN IIy
$R^1$-Cy-$Z^1$-Dit-$Z^1$-Phe-$CH_2CH_2$-A-CN IIz
$R^1$-Bi-$Z^1$-Phe-$Z^1$-Phe-$CH_2CH_2$-A-CN IIaa

$R^1$, $Z^1$ und A haben darin die angegebenen Bedeutungen.

Des weiteren umfassen Verbindungen der Formel II Pyridinethanderivate (Py) der Teilformel II am mit drei Ringen,

$R^1$-Cy-$CH_2CH_2$-Py-Phe-$R^2$ IIam

II br und II bt mit vier Ringen,

$R^1$-Cy-$Z^1$-Cy-$CH_2CH_2$-Phy-Phe-$R^2$ IIbr
$R^1$-Phe-Cy-$CH_2CH_2$-Py-Phe-$R^2$ IIbt

und II ch mit fünf Ringer,

$R^1$-Phe-$Z^1$-Cy-$Z^1$-Cy-$CH_2CH_2$-Py-Phe-$R^2$ IIch

$R^1$, $R^2$, A und $Z^1$ haben die angegebenen Bedeutungen.

Falls $A^2$ eine Einfachbindung darstellt, umfassen die Verbindungen der Formel II auch folgende Verbindungen der Teilformel IIcm,

$R^{1*}$-$CH_2CH_2$-A-CN IIcm

worin $R^{1*}$ die für $R^1$ angegebene Bedeutung hat, mit der Maßgabe, daß ein chirales C-Atom enthalten ist und A 1,4-Phenylen bedeutet, welches unsubstituiert oder durch F, Cl oder CN substituiert vorliegen kann.

Des weiteren umfassen die Verbindungen der Formel II denn auch die Verbindungen der Teilformel IIcq

$R^1$-$CH_2CH_2$-Py-Phe-$R^2$ IIcq

worin $R^1$

Py und $R^2$ die angegebenen Bedeutungen haben.

Die Verbindungen der Formel II sowie der vorstehenden Teilformeln sind teilweise bekannt. Sie umfassen jedoch auch neue Verbindungen der Form IV, die ebenfalls Gegenstand der Erfindung sind (für die Vertragsstaaten FR und IT),

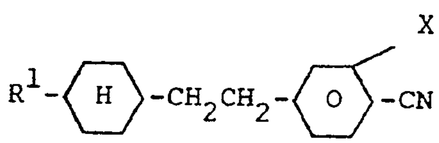

IV

worin

$R^1$ H, eine Alkylgruppe mit 1-12 C-Atomen bedeutet, die geradkettig oder verzweigt sein kann, worin auch eine oder mehrere $CH_2$-Gruppen durch O-Atome und/ oder -CO-Gruppen und/oder -O-CO-und/oder -CO-O-und/oder -CH=CH-Gruppen ersetzt sein können, wobei zwei Heteroatome nicht direkt miteinander verknüpft sind,

und

X′ F, Cl oder CN bedeutet.

Für $R^1$ kommen darin die bei Formel II angegeben Bedeutungen in Farge. X′ bedeutet vorzugsweise Fluor.

Die Verbindungen der formel IV können nach dem erfindungsgemäßen Verfahren hergestellt werden, und zwar z. B. durch Umsetzung der entsprechenden Cyclohexylmethyljodide mit dem durch X′ substituierten Tolunitril.

Die Verbindungen der Formeln II und IV können als Flüssigkristallmaterialien selbst und/oder als Ausgangsmaterialien für die Synthese von flüssigkristallinen Substanzen verwendet werden.

Beispielsweise können die Benzonitrile zu den entsprechenden Benzoesäuren verseift werden. Daraus lassen sich leicht entsprechende Ester herstellen, nach bekannten Veresterungsverfahren. Des weiteren besteht die Möglichkeit, die Benzoesäuren durch Hydrierung in die entsprechenden Cyclohexancarbonsäuren

umzuwandeln, die wiederum verestert werden können.

Aus den Cyclohexancarbonsäuren können die Nitrile hergestellt werden und anschließend der Cyclohexanring in 4-Stellung alkyliert werden. Verbindungen der Formel II und IV eignen sich auch zur Herstellung von Pyrimidinderivaten, indem man das Nitril in das entsprechende Amidin überführt, welches zum Pyrimidinring cyclisiert werden kann.

Zur Herstellung von Dioxanderivaten sind die Ethanderivate der Formeln II und IV ebenfalls geeignet. Dabei wird das

Nitrilderivat beispielsweise mit Diisobutylaluminiumhydrid in den entsprechenden Aldehyd überführt und anschließend zum Dioxanring kondensiert.

Des weiteren können die Nitrilgruppen über Grignard-Reaktion und Reduktion in Alkylreste überführt werden.

Das erfindungsgemäße Verfahren liefert also über einen präparativ einfachen Weg Ethanderivate z. B. mit endständiger CN-Gruppe, die wiederum als wichtige Zwischenprodukte eine Vielzahl von Synthesemöglichkeiten eröffnen.

Folgende Verbindungen sind beispielsweise nach dem erfindungsgemäßen Verfahren herstellbar:

2-(4-Butylphenyl)-5-propylpyridin
2-(4-Pentylphenyl)-5-propylpyridin
2-(4-Ethylphenyl)-5-propylpyridin
2-(4-Propylphenyl)-5-propylpyridin
2-(4-Ethylphenyl)-5-ethylpyridin
2-(4-Propylphenyl)-5-ethylpyridin
2-(4-Butylphenyl)-5-ethylpyridin
2-(4-Pentylphenyl)-5-ethylpyridin
2-[4-(3-Methylpentyl)phenyl]-5-ethylpyridin
2-(4-Ethylphenyl)-5-butylpyridin
2-(4-Propylphenyl)-5-butylpyridin
2-(4-Butylphenyl)-5-butylpyridin
2-(4-Pentylphenyl)-5-butylpyridin
2-(4-Ethylphenyl)-5-pentylpyridin
2-(4-Propylphenyl)-5-pentylpyridin
2-(4-Butylphenyl)-5-pentylpyridin
1-[trans-4-(4-Ethylphenyl)cyclohexyl]-2-(4-cyanophenyl)ethan
1-[trans-4-(4-Propylphenyl)cyclohexyl]-2-(4-cyanophenyl)ethan
1-[trans-4-(4-Butylphenyl)cyclohexyl]-2-(4-cyanophenyl)ethan
1-[trans-4-(4-Pentylphenyl)cyclohexyl]-2-(4-cyanophenyl)ethan
1-[trans-4-(4-Hexylphenyl)cyclohexyl]-2-(4-cyanophenyl)ethan
2-(p-Ethylphenyl)-5-octylpyridin
2-(p-Ethylphenyl)-5-heptylpyridin
2-(p-Ethylphenyl)-5-hexylpyridin
2-(p-Ethylphenyl)-5-pentylpyridin
2-(p-Propylphenyl)-5-octylpyridin
2-(p-Propylphenyl)-5-heptylpyridin
2-(p-Propylphenyl)-5-hexylpyridin
2-(p-Butylphenyl)-5-pentylpyridin
2-(p-Butylphenyl)-5-hexylpyridin
2-(p-Pentylphenyl)-5-pentylpyridin

Die folgenden Beispiele sollen die Erfindung erläutern, ohne sie zu begrenzen. Vor- und nachstehend bedeuten Prozentangaben Gewichtsprozent; alle Temperaturangaben sind in Grad Celsius angegeben. "Übliche Aufarbeitung" bedeutet: man gibt Wasser hinzu, extrahiert mit Methylenchlorid, trennt ab, trocknet die organische Phase, dampft ein und reinigt das Produkt durch Kristallisation und/oder Chromatographie. C-N bedeutet Übergang von kristallin zu nematisch und N-I von nematisch zu isotrop, Fp. = Schmelzpunkt und Kp. = Klärpunkt.

## Beispiel 1

Unter Ausschluß von Luftsauerstoff und Feuchtigkeit werden zu 300 ml THF bei -70° 750 ml einer 1,6 m Lösung von Butyllithium in Hexan, 121,5 g Diisopropylamin, 288 ml 1,3-Dimethyltetrahydro-2(1H)-pyrimidinon (DMPU) und 150 g Tolunitril in 750 ml THF zugegeben. Nach 20 Minuten gibt man ebenfalls bei -70° 495 g

trans-4-n-Pentylcyclohexylmethyljodid (herstellbar aus trans-4-n-Pentylcyclohexancarbonsäure durch Reduktion mit $LiAlH_4$ und Überführung des Alkohols in das entsprechende Sulfonat (Mesylat oder Tosylat) mit anschließender Finkelstein-Reaktion) in 750 ml THF zu. Unter Erwärmung auf Raumtemperatur wird noch 14 Stunden gerührt und anschließend wie üblich aufgearbeitet. Die leichtflüchtigen Anteile des Rohproduktes werden im Vakuum bis ca. 120 °C abdestilliert. Man erhält 1-(trans-4-n Pentylcyclohexyl)-2-(4-cyanophenyl)-ethan durch Umkristallisation des Destillationsrückstandes.

Analog werden hergestellt:

1-(trans-4-Ethylcyclohexyl)-2-(4-cyanophenyl)ethan
1-(trans-4-Propylcyclohexyl)-2-(4-cyanophenyl)ethan
1-(trans-4-Butylcyclohexyl)-2-(4-cyanophenyl)ethan
1-(trans-4-Hexylcyclohexyl)-2-(4-cyanophenyl)ethan
1-(trans-4-Heptylcyclohexyl)-2-(4-cyanophenyl)ethan
1-(trans-4-Octylcyclohexyl)-2-(4-cyanophenyl)ethan
1-(trans-4-Nonylcyclohexyl)-2-(4-cyanophenyl)ethan
1-(trans-4-Decylcyclohexyl)-2-(4-cyanophenyl)ethan
1-(trans-4-Ethylcyclohexyl)-2-(2-fluor-4-cyanophenyl)ethan
1-(trans-4-Propylcyclohexyl)-2-(2-fluor-4-cyanophenyl)ethan
1-(trans-4-Butylcyclohexyl)-2-(2-fluor-4-cyanophenyl)ethan
1-(trans-4-Pentylcyclohexyl)-2-(2-fluor-4-cyanophenyl)ethan, C-N 45°, N-I 54°
1-(trans-4-Hexylcyclohexyl)-2-(2-fluor-4-cyanophenyl)ethan
1-(trans-4-Heptylcyclohexyl)-2-(2-fluor-4-cyanophenyl)ethan, C-N 43,5°, N-I 56°
1-(trans-4-Octylcyclohexyl)-2-(2-fluor-4-cyanophenyl)ethan
1-(trans-4-Nonylcyclohexyl)-2-(2-fluor-4-cyanophenyl)ethan
1-(trans-4-Decylcyclohexyl)-2-(2-fluor-4-cyanophenyl)ethan
1-(trans-4-(trans-4-Ethylcyclohexyl)cyclohexyl)-2-(4-cyanophenyl)ethan
1-[trans-4-(trans-4-Propylcyclohexyl)cyclohexyl)-2-(4-cyanophenyl)ethan
1-[trans-4-(trans-4-Butylcyclohexyl)cyclohexyl)-2-(4-cyanophenyl)ethan
1-[trans-4-(trans-4-Pentylcyclohexyl)cyclohexyl)-2-(4-cyanophenyl)ethan
1-[trans-4-(trans-4-Hexylcyclohexyl)cyclohexyl)-2-(4-cyanophenyl)ethan
1-[trans-4-(trans-4-Heptylcyclohexyl-)cyclohexyl)-2-(4-cyanophenyl)ethan
1-[trans-4-(trans-4-Octylcyclohexyl)cyclohexyl)-2-(4-cyanophenyl)ethan
1-[trans-4-(trans-4-Nonylcyclohexyl)cyclohexyl)-2-(4-cyanophenyl)ethan
1-[trans-4-(trans-4-Decylcyclohexyl)cyclohexyl)-2-(4-cyanophenyl)ethan
1-[trans-4-(trans-4-Ethylcyclohexyl)cyclohexyl)-2-(2-fluor-4-cyanophenyl)ethan
1-[trans-4-(trans-4-Propylcyclohexyl)cyclohexyl)-2-(2-fluor-4-cyanophenyl)ethan
1-[trans-4-(trans-4-Butylcyclohexyl)cyclohexyl)-2-(2-fluor-4-cyanophenyl)ethan
1-[trans-4-(trans-4-Pentylcyclohexyl)cyclohexyl)-2-(2-fluor-4-cyanophenyl)ethan, C-N 115°, N-I 187°
1-[trans-4-(trans-4-Hexylcyclohexyl)cyclohexyl)-2-(2-fluor-4-cyanophenyl)ethan
1-[trans-4-(trans-4-Heptylcyclohexyl)cyclohexyl)-2-(2-fluor-4-cyanophenyl)ethan
1-[trans-4-(trans-4-Octylcyclohexyl)cyclohexyl)-2-(2-fluor-4-cyanophenyl)ethan
1-[trans-4-(trans-4-Nonylcyclohexyl)cyclohexyl)-2-(2-fluor-4-cyanophenyl)ethan
1-[trans-4-(trans-4-Decylcyclohexyl)cyclohexyl)-2-(2-fluor-4-cyanophenyl)ethan
1-[trans-4-(trans-4-Exylcyclohexyl)cyclohexyl)-2-(3-fluor-4-cyanophenyl)ethan
1-[trans-4-(trans-4-Propylcyclohexyl)cyclohexyl)-2-(3-fluor-4-cyanophenyl)ethan
1-[trans-4-(trans-4-Butylcyclohexyl)cyclohexyl)-2-(3-fluor-4-cyanophenyl)ethan, C-N 59°, N-I 164,2°
1-[trans-4-(trans-4-Pentylcyclohexyl)cyclohexyl)-2-(3-fluor-4-cyanophenyl)ethan
1-[trans-4-(trans-4-Hexylcyclohexyl)cyclohexyl)-2-(3-fluor-4-cyanophenyl)ethan
1-[trans-4-(trans-4-Heptylcyclohexyl)cyclohexyl)-2-(3-fluor-4-cyanophenyl)ethan
1-[trans-4-(trans-4-Octylcyclohexyl)cyclohexyl)-2-(3-fluor-4-cyanophenyl)ethan
1-[trans-4-(trans-4-Nonylcyclohexyl)cyclohexyl)-2-(3-fluor-4-cyanophenyl)ethan
1-[trans-4-(trans-4-Decylcyclohexyl)cyclohexyl)-2-(3-fluor-4-cyanophenyl)ethan.

Beispiel 2

Unter Ausschluß von Sauerstoff und Feuchtigkeit werden zu 300 ml THF bei -70° 750 ml einer 1,6 m Lösung von Butyllithium in Hexan, 121,5 g Diisopropylamin, 288 ml 1,3-Dimethyltetrahydro-2(1H)-pyrimidinon und 175 g 3-Fluor-Tolunitril in 750 ml THF zugegeben. Nach 20 Minuten gibt man ebenfalls bei -70° 495 g trans-4-n-Pentylcyclohexylmethyljodid (herstellbar aus trans-4-n-Pentylcyclohexancarbonsäure durch Reduktion mit

$LiAlH_4$ und Überführung des Alkohols in das entsprechende Sulfonat (Mesylat oder Tosylat) mit anschließender Finkelstein-Reaktion) in 750 ml THF zu. Man läßt auf Raumtemperatur kommen und rührt noch 14 Stunden. Nach üblicher Aufarbeitung erhält man 1-(trans-4-n-Pentylcyclohexyl)-2-(3-fluor-4-cyanophenyl)ethan.

Analog werden hergestellt:

1-(trans-4-Ethylcyclohexyl)-2-(3-fluor-4-cyanophenyl)ethan
1-(trans-4-Propylcyclohexyl)-2-(3-fluor-4-cyanophenyl)ethan, C-I 19,2° (N-I 7,2°)
1-(trans-4-Butylcyclohexyl)-2-(3-fluor-4-cyanophenyl)ethan
1-(trans-4-Hexylcyclohexyl)-2-(3-fluor-4-cyanophenyl)ethan
1-(trans-4-Heptylcyclohexyl)-2-(3-fluor-4-cyanophenyl)ethan
1-(trans-4-Octylcyclohexyl)-2-(3-fluor-4-cyanophenyl)ethan
1-(trans-4-Nonylcyclohexyl)-2-(3-fluor-4-cyanophenyl)ethan
1-(trans-4-Decylcyclohexyl)-2-(3-fluor-4-cyanophenyl)ethan.

Beispiel 3

Zu 50 ml THF werden unter Stickstoff 66,3 ml Butyllithium bei -15° zugegeben. Dann werden bei -70° 10,7 g Diisopropylamin und 13,6 g DMPU zugegeben. Anschließend gibt man bei -40° 19,2 g 2-(p-Methoxyphenyl)-5-methylpyridin in 50 ml THF und danach 17,6 g 1-Bromheptan zu. Man rührt 2 Stunden bei Raumtemperatur, gibt 100 ml 1 N HCl zu, rührt und alkalisiert dann mit NaOH. Die organische Phase wird abgetrennt, gewaschen und getrocknet. Nach Umkristallisation aus Methanol enthält man 2-(p-Methoxyphenyl)-5-octylpyridin.

Analog werden hingestellt:

2-(p-Methoxyphenyl)-5-octylpyridin
2-(p-Methoxyphenyl)-5-heptylpyridin
2-(p-Methoxyphenyl)-5-hexylpyridin
2-(p-Methoxyphenyl)-5-pentylpyridin
2-(p-Methoxyphenyl)-5-butylpyridin
2-(p-Methoxyphenyl)-5-propylpyridin
2-(p-Ethoxyphenyl)-5-octylpyridin
2-(p-Ethoxyphenyl)-5-heptylpyridin
2-(p-Ethoxyphenyl)-5-hexylpyridin
2-(p-Ethoxyphenyl)-5-pentylpyridin
2-(p-Ethoxyphenyl)-5-butylpyridin
2-(p-Ethoxyphenyl)-5-propylpyridin
2-(p-Propoxyphenyl)-5-octylpyridin
2-(p-Propoxyphenyl)-5-heptylpyridin
2-(p-Propoxyphenyl)-5-hexylpyridin
2-(p-Propoxyphenyl)-5-pentylpyridin
2-(p-Propoxyphenyl)-5-butylpyridin
2-(p-Propoxyphenyl)-5-propylpyridin
2-(p-Butoxyphenyl)-5-pentylpyridin
2-(p-Butoxyphenyl)-5-hexylpyridin
2-(p-Butoxyphenyl)-5-heptylpyridin
2-(p-Butoxyphenyl)-5-octylpyridin
2-(p-Butoxyphenyl)-5-butylpyridin
2-(p-Butoxyphenyl)-5-propylpyridin

## Patentansprüche

1. Verfahren zur Herstellung von Ethanderivaten der Formel II

$$R^1\text{-}(A^1\text{-}Z^1)_n\text{-}A^2\text{-}CH_2CH_2\text{-}A\text{-}Q \qquad II$$

worin

Q CN oder, falls A Pyridin-2,5-diyl ist, p-$R^2$-Phenyl,

$R^1$ und $R^2$ jeweils unabhängig voneinander H, eine Alkylgruppe mit 1 - 12 C-Atomen bedeutet, die geradkettig oder verzweigt sein kann, worin auch eine oder mehrere $CH_2$-Gruppen durch O-Atome und/oder -CO-Gruppen und/oder -O-CO- und/oder -CO-O- und/oder -CH=CH-Gruppen ersetzt sein

können, wobei zwei Heteroatome nicht direkt miteinander verknüpft sind,

| | |
|---|---|
| A | unsubstituiertes oder ein oder mehrmals durch F, Cl oder CN substituiertes 1,4-Phenylen, oder Pyridin-2,5-diyl, |
| $A^1$ | eine unsubstituierte oder durch F- und/oder Cl-Atome und/oder $CH_3$- und/oder CN-Gruppen substituierte 1,4-Cyclohexylengruppe, worin auch eine oder zwei nicht benachbarte $CH_2$-Gruppen durch O-Atome und/oder S-Atome ersetzt sein können, 1,4-Bicyclo(2,2,2)octylengruppe oder 1,4-Phenylengruppe, worin auch eine oder mehrere CH-Gruppen durch N ersetzt sein können, |
| $A^2$ | eine unsubstituierte oder durch F- und/oder Cl-Atome und/oder $CH_3$- und/oder CN-Gruppen substituierte 1,4-Cyclohexylengruppe, oder im Falle A Pyridin-2,5-diyl, $A^2$ auch eine Einfachbindung, |
| $Z^1$ | -CO-O-, -O-CO-, $-CH_2CH_2-$, $-OCH_2-$, $-CH_2O-$ oder eine Einfachbindung, |
| und | |
| n | 0, 1 oder 2 |

bedeutet, mit der Maßgabe, daß n = 0 ist, wenn $A^2$ eine Einfachbindung bedeutet, dadurch gekennzeichnet, daß man eine reaktionsfähige Methylenverbindung der Formel III

$$R^1\text{-}(A^1\text{-}Z^1)_n\text{-}A^2\text{-}CH_2\text{-}Y \qquad III$$

worin $R^1$, $A^1$, $Z^1$, n und $A^2$ die angegebene Bedeutung haben und Y ein Halogen darstellt, mit einer Verbindung der Formel I

$$CH_3\text{-}A\text{-}Q$$

worin A und Q die angegebene Bedeutung haben, in Gegenwart einer starken Base umsetzt.

**Patentansprüche für folgende Vertragsstaaten: FR und IT**

1. Verfahren zur Herstellung von Ethanderivaten der Formel II

$$R^1\text{-}(A^1\text{-}Z^1)_n\text{-}A^2\text{-}CH_2CH_2\text{-}A\text{-}Q \qquad II$$

worin

| | |
|---|---|
| Q | CN oder, falls A Pyridin-2,5-diyl ist, p-$R^2$-Phenyl, |
| $R^1$ und $R^2$ | jeweils unabhängig voneinander H, eine Alkylgruppe mit 1 - 12 C-Atomen bedeutet, die geradkettig oder verzweigt sein kann, worin auch eine oder mehrere $CH_2$-Gruppen durch O-Atome und/oder -CO-Gruppen und/oder -O-CO- und/oder -CO-O- und/oder -CH=CH-Gruppen ersetzt sein können, wobei zwei Heteroatome nicht direkt miteinander verknüpft sind, |
| A | unsubstituiertes oder ein oder mehrmals durch F, Cl oder CN substituiertes 1,4-Phenylen, oder Pyridin-2,5-diyl, |
| $A^1$ | eine unsubstituierte oder durch F- und/oder Cl-Atome und/oder $CH_3$- und/oder CN-Gruppen substituierte 1,4-Cyclohexylengruppe, worin auch eine oder zwei nicht benachbarte $CH_2$-Gruppen durch O-Atome und/oder S-Atome ersetzt sein können, 1,4-Bicyclo(2,2,2)octylengruppe oder 1,4-Phenylengruppe, worin auch eine oder mehrere CH-Gruppen durch N ersetzt sein können, |
| $A^2$ | eine unsubstituierte oder durch F- und/oder Cl-Atome und/oder $CH_3$- und/oder CN-Gruppen substituierte 1,4-Cyclohexylengruppe, oder im Falle A Pyridin-2,5-diyl, $A^2$ auch eine Einfachbindung, |
| $Z^1$ | -CO-O-, -O-CO-, $-CH_2CH_2-$, $-OCH_2-$, $-CH_2O-$ oder eine Einfachbindung, |
| und | |
| n | 0, 1 oder 2 |

bedeutet, mit der Maßgabe, daß n = 0 ist, wenn $A^2$ eine Einfachbindung bedeutet, dadurch gekennzeichnet, daß man eine reaktionsfähige Methylenverbindung der Formel III

$$R^1\text{-}(A^1\text{-}Z^1)_n\text{-}A^2\text{-}CH_2\text{-}Y \qquad III$$

worin $R^1$, $A^1$, $Z^1$, n und $A^2$ die angegebene Bedeutung haben und Y ein Halogen darstellt, mit einer Verbindung der Formel I

$$CH_3\text{-}A\text{-}Q$$

worin A und Q die angegebene Bedeutung haben, in Gegenwart einer starken Base umsetzt.

2. Verbindungen der Formel IV

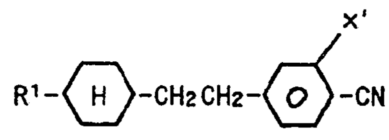

IV

worin

$R^1$ H, eine Alkylgruppe mit 1 - 12 C-Atomen bedeutet, die geradkettig oder verzweigt sein kann, worin auch eine oder mehrere $CH_2$-Gruppen durch O-Atome und/oder -CO-Gruppen und/oder -O-CO- und/oder -CO-O- und/oder -CH=CH-Gruppen ersetzt sein können, wobei zwei Heteroatome nicht direkt miteinander verknüpft sind

und

X' F, Cl oder CN bedeutet.

## Claims

1. Process for the preparation of ethane derivatives of the formula II

$$R^1\text{-}(A^1\text{-}Z^1)_n\text{-}A^2\text{-}CH_2CH_2\text{-}A\text{-}Q \qquad II$$

wherein

| | |
|---|---|
| Q | is CN, or, if A is pyridine-2,5-diyl, Q is p-$R^2$-phenyl, |
| $R^1$ and $R^2$ | independently of one another are each H or an alkyl group which has 1-12 C atoms and which can be linear or branched and in which one or more $CH_2$ groups can also be replaced by O atoms and/or -CO- groups and/or -O-CO- and/or -CO-O-and/or -CH=CH- groups, but two hetero atoms are not directly attached to one another, |
| A | is 1,4-phenylene which is unsubstituted or is monosubstituted or polysubstituted by F, Cl or CN or pyridine-2,5-diyl, |
| $A^1$ | is a 1,4-cyclohexylene group which is unsubstituted or substituted by F and/or Cl atoms and/or $CH_3$ and/or CN groups, and in which one or two non-adjacent $CH_2$ groups can also be replaced by O atoms and/or S atoms, or a 1,4-bicyclo-(2,2,2)octylene group or 1,4-phenylene group in which one or more CH groups can be replaced by N, |
| $A^2$ | is a 1,4-cyclohexylene group which is unsubstituted or substituted by F and/or Cl atom and/or $CH_3$ and/or CN groups, or, in the case where A is pyridine-2,5-diyl, $A^2$ is also a single bond, |
| $Z^1$ | is a -CO-O-, -O-CO-, $-CH_2CH_2-$, $-OCH_2-$, $-CH_2O-$ or a single bond, and |
| n | is 0, 1 or 2, |

subject to the proviso that n is O if $A^2$ is a single bond, characterised in that a reactive methylene compound of the formula III

$$R^1\text{-}(A^1\text{-}Z^1)_n\text{-}A^2\text{-}CH_2\text{-}Y \qquad III$$

wherein $R^1$, $A^1$, $Z^1$, n and $A^2$ have the meaning indicated and Y is a halogen, is reacted, in the presence of a strong base, with a compound of the formula I

$$CH_3\text{-}A\text{-}Q$$

wherein A and Q have the meaning indicated.

## Claims for the following Contracting States: FR, IT

1. Process for the preparation of ethane derivatives of the formula II

$$R^1\text{-}(A^1\text{-}Z^1)_n\text{-}A^2\text{-}CH_2CH_2\text{-}A\text{-}Q \qquad II$$

wherein

| | |
|---|---|
| Q | is CN, or, if A is pyridine-2,5-diyl, Q is p-$R^2$-phenyl, |
| $R^1$ and $R^2$ | independently of one another are each H or an alkyl group which has 1-12 C atoms and which can be linear or branched and in which one or more $CH_2$ groups can also be replaced by O atoms and/or -CO- groups and/or -O-CO- and/or -CO-O-and/or -CH=CH- groups, but two hetero atoms are not directly attached to one another, |
| A | is 1,4-phenylene which is unsubstituted or is monosubstituted or polysubstituted by F, Cl or CN or pyridine-2,5-diyl, |
| $A^1$ | is a 1,4-cyclohexylene group which is unsubstituted or substituted by F and/or Cl atoms and/or $CH_3$ and/or CN groups, and in which one or two non-adjacent $CH_2$ groups can also be replaced |

| | |
|---|---|
| | by O atoms and/or S atoms, or a 1,4-bicyclo-(2,2,2)octylene group or 1,4-phenylene group in which one or more CH groups can be replaced by N, |
| $A^2$ | is a 1,4-cyclohexylene group which is unsubstituted or substituted by F and/or Cl atoms and/or $CH_3$ and/or CN groups, or, in the case where A is pyridine-2,5-diyl, $A^2$ is also a single bond, |
| $Z^1$ | is a -CO-O-, -O-CO-, $-CH_2CH_2-$, $-OCH_2-$, $-CH_2O-$ or a single bond, and |
| n | is 0, 1 or 2, |

subject to the proviso that n is O if $A^2$ is a single bond, characterised in that a reactive methylene compound of the formula III

$$R^1-(A^1-Z^1)\ n-A^2-CH_2-Y \qquad III$$

wherein $R^1$, $A^1$, $Z^1$, n and $A^2$ have the meaning indicated and Y is a halogen, is reacted, in the presence of a strong base, with a compound of the formula I

$$CH_3-A-Q$$

wherein A and Q have the meaning indicated.

2. Compounds of the formula IV

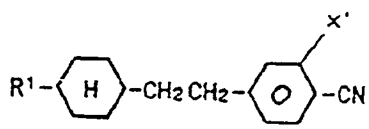

IV

wherein

| | |
|---|---|
| $R^1$ | is H or an alkyl group which has 1-12 C atoms and which can be linear or branched and in which one or more $CH_2$ groups can also be replaced by O atoms and/or -CO- groups and/or -O-CO- and/or -CO-O- and/or -CH=CH- groups, but two hetero atoms are not directly attached to one another, and |
| X' | is F, CL or CN. |

## Revendications

1. Procédé pour la préparation des dérivés d'éthane de formule II

$$R^1-(A^1-Z^1)_n-A^2-CH_2CH_2-A-Q \qquad II$$

où

| | |
|---|---|
| R | représente CN ou, dans le cas où A est le pyridin-2,5-diyle, p-$R^2$-phényle, |
| $R^1$ et $R^2$ | représentent, indépendamment l'un de l'autre, H, un groupe alkyle ayant 1 à 12 atomes de C, pouvant être linéaire ou ramifié, un ou plusieurs groupes $CH_2$ pouvant être également remplacer par des atomes d'O et/ou des groupes -CO- et/ou des groupes -O-OCO-et/ou -CO-O- et/ou -CH=CH-, deux hétéroatomes ne pouvant être directement liés l'un à l'autre, |
| A | représente le 1,4-phénylène non substitué ou une ou plusieurs fois substitué par F, Cl ou CN ou le pyridin-2,5-diyle, |
| $A^1$ | représente un groupe 1,4-cyclohexylène non substitué ou substitué par des atomes de F, Cl et/ou Cl et/ou des groupes $CH_3$ et/ou CN, un ou deux groupes $CH_2$ non voisins pouvant être également remplacés par des atomes d'O et/ou de S, un groupe 1,4-bicyclo(2,2,2)octylène ou un groupe 1,4-phénylène, un ou plusieurs groupes CH pouvant être également remplacé par N, |
| $A^2$ | représente un groupe 1,4-cyclohexylène non substitué ou substitué par des atomes de F et/ou de Cl et/ou des groupes $CH_3$ et/ou CN ou, dans le cas où A est le pyridin-2,5-diyle, $A^2$ peut également représenter une liaison simple, |
| $Z^1$ | représente -CO-O-, -O-CO-, $-CH_2CH_2-$, $-OCH_2-$, $-CH_2O-$ ou une liaison simple, |
| | et |
| n | égal à 0, 1 ou 2, sous réserve que n=0, lorsque $A^2$ représente une liaison simple, caractérisé en ce que l'on fait réagir un composé méthylénique réactif de formule III |

$$R^1-(A^1-Z^1)_n-A^2-CH_2-Y \qquad III$$

où $R^1$, $A^1$, $Z^1$, n et $A^2$ ont la signification indiquée et Y représente un halogène, avec un composé de formule I

$$CH_3-A-Q$$

où A et Q ont la signification indiquée, en présence d'une base forte.

**Revendications pour les Etats contractants suivants: FR, IT**

1. Procédé pour la préparation des dérivés d'éthane de formule II

$$R^1\text{-}(A^1\text{-}Z^1)_n\text{-}A^2\text{-}CH_2CH_2\text{-}A\text{-}Q \qquad II$$

où

R représente CN ou, dans le cas où A est le pyridin-2,5-diyle, p-$R^2$-phényle,

$R^1$ et $R^2$ représentent, indépendamment l'un de l'autre, H, un groupe alkyle ayant 1 à 12 atomes de C, pouvant être linéaire ou ramifié, un ou plusieurs groupes $CH_2$ pouvant être également remplacer par des atomes d'O et/ou des groupes -CO- et/ou des groupes -O-OCO- et/ou -CO-O- et/ou -CH=CH-, deux hétéroatomes ne pouvant être directement liés l'un à l'autre,

A représente le 1,4-phénylène non substitué ou une ou plusieurs fois substitué par F, Cl ou CN ou le pyridin-2,5-diyle,

$A^1$ représente un groupe 1,4-cyclohexylène non substitué ou substitué par des atomes de F, Cl et/ou Cl et/ou des groupes $CH_3$ et/ou CN, un ou deux groupes $CH_2$ non voisins pouvant être également remplacés par des atomes d'O et/ou de S, un groupe 1,4-bicyclo(2,2,2)octylène ou un groupe 1,4-phènylène, un ou plusieurs groupes CH pouvant être également remplacé par N,

$A^2$ représente un groupe 1,4-cyclohexylène non substitué ou substitué par des atomes de F et/ou de Cl et/ou des groupes $CH_3$ et/ou CN ou, dans le cas où A est le pyridin-2,5-diyle, $A^2$ peut également représenter une liaison simple,

$Z^1$ représente -CO-O-, -O-CO-, $-CH_2CH_2-$, $-OCH_2-$, $-CH_2O-$ ou une liaison simple, et

n égal à 0, 1 ou 2,

sous réserve que n=0, lorsque $A^2$ représente une liaison simple, caractérisé en ce que l'on fait réagir un composé méthylénique réactif de formule III :

$$R^1\text{-}(A^1\text{-}Z^1)_n\text{-}A^2\text{-}CH_2\text{-}Y \qquad III$$

où $R^1$, $A^1$, $Z^1$, n et $A^2$ ont la signification indiquée et Y représente un halogène, avec un composé de formule I

$$CH_3\text{-}A\text{-}Q$$

où A et Q ont la signification indiquée, en présence d'une base forte.

2. Composés de formule IV

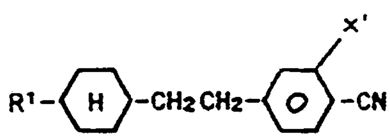

IV

où

$R^1$ représente H, un groupe alkyle ayant 1 à 12 atomes de C, pouvant être à chaîne linéaire ou ramifiée, un ou plusieurs groupes $CH_2$ pouvant être également remplacés par des atomes d'O et/ou des groupes -CO- et/ou des groupes -O-CO- et/ou des groupes -O-CO-et/ou des groupes -CH=CH-, deux hétéroatomes ne pouvant être directement liés l'un à l'autre et

X' représente F, Cl ou CN.